# EUROPEAN PATENT SPECIFICATION

(11) **EP 2 983 616 B1**
(45) Date of publication and mention of the grant of the patent: **03.10.2018**
(21) Application number: 14783373.5
(22) Date of filing: 08.04.2014
(51) Int. Cl.: A61L 27/36

(54) **METHOD AND COMPOSITION FOR TREATING INFLAMMATORY BOWEL DISEASE WITHOUT COLECTOMY**
VERFAHREN UND ZUSAMMENSETZUNG ZUR BEHANDLUNG ENTZÜNDLICHER DARMERKRANKUNGEN OHNE KOLEKTOMIE
PROCÉDÉ ET COMPOSITION POUR TRAITER UNE MALADIE INTESTINALE INFLAMMATOIRE SANS COLECTOMIE

(30) Priority: 08.04.2013 US 201361809606 P; 05.07.2013 US 201361843286 P; 07.11.2013 US 201361901237 P
(43) Date of publication of application: 17.02.2016
(73) Proprietor: Regentys Corporation, Miami Lakes, FL 33015 (US); University of Pittsburgh - Of the Commonwealth System of Higher Education, Pittsburgh, PA 15260 (US)
(72) Inventor: RAMER, Marc, Weston, FL 33326 (US); BADYLAK, Stephen, F., Pittsburgh, PA 15213 (US); KEANE, Timothy, Wellsboro, PA 16901 (US)
(74) Representative: Schulz Junghans Patentanwälte PartGmbB
(86) International application number: PCT/US2014/033365
(87) International publication number: WO 2014/168964

(56) References cited:
- WO-A2-2012/064606
- US-A- 5 885 619
- US-A1- 2009 204 228
- US-A1- 2013 058 991
- US-B2- 6 890 564
- QIQI ZHOU ET AL: "Localized colonic stem cell transplantation enhances tissue regeneration in murine colitis", JOURNAL OF CELLULAR AND MOLECULAR MEDICINE, vol. 16, no. 8, 1 August 2012 (2012-08-01) , pages 1900-1915, XP055288868, RO ISSN: 1582-1838, DOI: 10.1111/j.1582-4934.2011.01485.x
- LANZONI ET AL.: 'Inflammatory bowel disease: Moving toward a stem cell -based therapy.' WORLD J GASTROENTEROL. vol. 14, no. 29, 07 August 2008, pages 4616 - 4626, XP055127973
- ZHOU ET AL.: 'Localized colonic stem cell transplantation enhances tissue regeneration in murine colitis.' J CELL MOL MED. vol. 16, no. 8, August 2012, pages 1900 - 1915, XP055288868

## Description

### BACKGROUND

Inflammatory Bowel Disease (IBD) consists of two independent diseases: Crohn's Disease (CD) and Ulcerative Colitis (UC), affecting 833,000 Americans.

While CD can manifest anywhere along the digestive tract (mouth to anus) and often affects the entire thickness of the digestive tract wall, UC is confined to the colon and rectum and affects only the mucosa (inner lining) of the wall.

Medical treatments, including use of anti-inflammatories that may have severe side effects, may fail in treating the symptoms of UC, and complete removal of the colon (colectomy) is the only known "cure" for UC when the patient is refractory to all other medical treatments. Colectomy is a well-established but major surgery, often requiring an ileostomy to facilitate waste removal.

Alternatives to first lines of treatment (medications with potentially harmful side effects) and second lines of treatment (colectomy) are needed for treating and curing IBD, including UC.

The trend toward minimally invasive surgical procedures has prompted the development of artificial scaffolds useful for tissue regeneration. Purified collagen, gelatin, autologous fat, hyaluronic acid, and synthetic materials have been clinically used as injectable scaffolds in regenerative medicine for the treatment of urinary incontinence, reflux disease, laryngeal pathologies, and neonatal cardiomyocytes. However, overly-purified, chemically modified or synthetic materials can lead to adverse immune responses by the host and limit cell migration into the matrix.

Naturally occurring extracellular matrix (ECM)-derived scaffolds possess many bioactive properties and have been used for the repair of a variety of tissues, including lower urinary tract structures, esophagus, cardiac tissue, and musculotendonous structures. However, many of these scaffolds are derived from non-human tissue, and none have been described for use in the treatment or regeneration of lower intestine tissue, such as colon tissue, and not specifically for the treatment of UC.

### SUMMARY OF THE INVENTION

The scope of the invention is defined by the claims. Any references in the description to methods of treatment refer to the compounds, pharmaceutical compositions and medicaments of the present invention for use in a method for treatment of the human (or animal) body by therapy.

The method of the invention as defined in claim 1 comprises treating damaged or diseased lower intestinal tissue by the steps of (1) optionally performing a mucosectomy on the diseased or damaged mucosal lining of the colon, and (2) seeding the mucosal lining of the colon with stem or progenitor cells, or stimulating stem and progenitor cell migration to the diseased or damaged colon tissue in a patient having symptoms of, or suffering from, disease or damage to the mucosal lining of the colon. A disease of the mucosal lining of the colon can be Inflammatory Bowel Disease (IBD), and the method can be used to treat, ameliorate, or cure IBD.

In one embodiment of the invention, stem cells or progenitor cells are stimulated to the site of the damaged or diseased colon tissue by introducing onto, or in contact with, the colon luminal wall an extracellular matrix (ECM) composition, e.g., an ECM graft or scaffold, in the form of a solid (sheet or tube) or fluid (solution, suspension, or gel).

In another embodiment of the invention, stem cells or progenitor cells are incorporated into the solid or fluid ECM composition, and the ECM composition comprising stem cells or progenitor cells can be delivered to the mucosal lining of the colon by delivery means appropriate for the composition as described herein. For example, a solid ECM can be endoscopically implanted onto the colon lining, or a fluid ECM can be infused, injected, or sprayed into the colon.

Once seeded or migrated to the site of damaged colon tissue, the stem cells or progenitor cells specialize, develop in situ, and replace the mucosal lining of the colon. The method can further include one or more of the additional steps of: (3) creating an ileostomy, and (4) providing Total Parenteral Nutrition (TPN) to the patient by feeding the patient intravenously, bypassing the digestive tract while the new mucosa forms and replaces the diseased mucosal lining.

When employed as part of the method of the invention, the mucosectomy step, which removes the diseased or damaged tissue prior to implantation, transplantation, or administration of the graft, can be carried out by tissue resection or ablation.

Resection or ablation methods include endoscopic or surgical mucosal resection (excision), endoscopic laser therapy, photodynamic therapy, endoscopic thermocoagulation using argon plasma coagulation, radiofrequency ablation, cryoablation, or the use of EDTA or other ablating agent such as saline which is preferably heated. These mucosectomy procedures are well documented in the art. Any one or more of the above techniques or procedures may be used in a method of the subject invention where a resection or ablation step is employed.

In one embodiment, the tissue graft composition can be an ECM scaffold formed as a solid sheet or tube which can be placed or secured onto the colon mucosa. A solid sheet or tube scaffold can be implanted or transplanted or, as recognized in the art, affixed, applied, or attached, at a desired site in a patient by mechanical or surgical means, including but not limited to sutures, staples, stents, or clips, or can be adhered to the mucosa by an acceptable adhesive, such as a pharmaceutically acceptable or therapeutically acceptable adhesive, including a bioadhesive. One preferred procedure for delivering an ECM composition to the colon can employ an endoscope, which can advantageously minimize invasive surgical procedures.

Alternatively, the ECM can be powdered or digested, and solubilized or suspended for presentation in the form of a fluid, such as a gel, solution, or suspension. A fluid composition can advantageously be administered by injecting, infusing, spraying, or the like, the gel, solution or suspension to the site being treated. For example, a fluidized ECM can be administered or applied by being injected, infused, or sprayed into the lumen of the colon, e.g., as an enema therapy whereby the fluid ECM coats the mucosal lining of the colon or, in the case of mucosectomized colon, coats the exposed lumen wall of the colon. Such administration can be repeated several times, including one or more times a day for a period of several days, weeks or months, up to about one year as determined by the treating physician monitoring for acceptable regrowth or replacement of viable or healthy tissue, as desired.

One preferred embodiment of a sprayable gel, solution or suspension of a fluidized ECM is to aerosolize the composition for administration by spraying. The aerosolized spray can be pumped from a source reservoir, through a cannula or other conduit provided in or with an endoscope manufactured or modified to deliver such fluidized ECM to the desired site or location.

Advantageously, stem cells or progenitor cells are known to migrate to the site of the ECM placement or administration and, over time, can replace the tissue with healthy or non-symptomatic cells and tissue having the normal properties and function of those cells or tissue. Thus, alternative embodiments of a composition of the invention include a solid ECM tube or sheet scaffold, alone; a solid ECM scaffold coated or embedded with stem or progenitor cells; a fluidized ECM (e.g., gel), alone; or a solid ECM scaffold additionally comprising fluidized ECM, with or without stem or progenitor cells coated, embedded, or mixed with the solid or fluid ECM.

It would also be understood that the ECM composition can be a hybrid, being derived in part from natural tissue and mixed or combined with a synthetic or natural polymer. It is preferable, but not required that a biodegradable polymer be used in an ECM composition of the invention comprising a synthetic or natural polymer.

In another embodiment of the invention, e.g., a solid sheet or tube ECM composition, can be formed or produced by use of three-dimensional (3-D) printer technology.

The composition can further include an additive for effecting a particular desired property to the ECM. For example, the ECM composition can include one or more drugs or biologics, such as an antibiotic, anti-inflammatory, immunosuppressant, monoclonal antibody, growth factor, or the like, providing a desired activity of the added drug or biologic. These additives can make the local environment conducive to favorable tissue remodeling. Other additives to the composition can include, for example a viscosity enhancing agent, or an agent which can initiate the formation of gel. It is generally accepted in the art that higher viscosity of a gel can provide improved adhesion properties of the gel. Accordingly, a viscosity enhancing agent can also serve to facilitate adhesion. Adhesive facilitators are well known in the art, but can further include blood components, such as blood coagulants or blood coagulation factors, as a viscosity enhancing agent or adhesion enhancing agent.

Also included as part of the invention is a method for treating diseased or damaged colon tissue using an ECM graft or scaffold as described herein, without prior resection or ablation. For example, in instances where disease or damage to the tissue creates a lesion, such as an exposed or bleeding surface of the tissue, resection of the tissue prior to implantation, transplantation, application, or administration of the ECM scaffold or graft composition may be omitted. Accordingly, one embodiment of the subject method comprises the steps of:
a) preparation of an ECM scaffold, graft, or composition using tissue selected from intestine, reproductive, integumentary, pancreatic, renal, circulatory, and respiratory, and
b) implanting, transplanting, applying or administering the ECM graft or scaffold composition onto tissue in a patient suffering from a disease or condition affecting the lower intestinal tract tissue in a patient suffering from disease, damage, or a condition affecting the intestinal tissue, wherein the intestinal tissue is not subjected to resection or ablation prior to the implantation, transplantation, application or administration of the ECM composition.

In a preferred embodiment, this method comprises treating damaged or diseased colon tissue exhibiting IBD, such as Ulcerative Colitis or Crohn's Disease; familial adenomatous polyposis; Hirschsprungs; stricture; proctitits; colon cancers; or fistula, and more preferably, treating disease or damage to colon mucosa tissue.

Another embodiment of the invention is a method for treating diseased or damaged tissue using a tissue-specific ECM (TS-ECM) scaffold as described herein, with or without prior resection or ablation. Accordingly, one embodiment of the subject method comprises the steps of:
a. optionally performing a mucosectomy on the damaged or diseased tissue;
b. preparing a TS-ECM graft or scaffold composition using tissue selected from intestine, reproductive(other than vaginal), integumentary, pancreatic, renal, circulatory, and respiratory, and
c. implanting, transplanting, or administering the TS-ECM scaffold or composition onto tissue in a patient suffering from a disease or condition affecting the same intestinal, reproductive (other than vaginal), integumentary, pancreatic, renal, circulatory, and respiratory tissue.

It would be understood that an ECM orTS-ECM graft, scaffold or composition of the invention can be a biocompatible porous, macroporous, or microporous matrix wherein stem cells or ECM gel, solution or suspension can be dispersed. In addition, an ECM orTS-ECM of the invention can be gelled.

Methods for preparing tube, sheet, and gelled, solubilized ECM compositions, useful as cell growth matrices or scaffolds used as grafts, are described in the art. Gel ECM compositions can be molded prior to implantation or administered to a patient in an un-gelled form prior to gelation where the composition gels in situ. These, and other methods of forming ECM grafts are described in, for example, US Patent No. 8,361,503.

In a preferred embodiment, a gel ECM composition is prepared according to one or more processes as described. For example, a gel ECM can be prepared by a method comprising:
i) comminuting an ECM,
ii) solubilizing intact, non-dialyzed or non-cross-linked ECM by digestion with an acid protease in an acidic solution to produce a digest solution,
iii) adjusting the pH of the digest solution to a pH between 7.2 and 7.8 to produce a neutralized digest solution, and
iv) gelling the solution at a temperature greater than 25 °C.

In another embodiment the method of preparing an ECM graft or scaffold composition further includes ultrasonicating the scaffold. A further method of the invention comprises attaching a TS-ECM scaffold to tissue of a patient wherein the surface of the scaffold comprises a gelled solubilized ECM embedded or coated with a patient's cells, e.g., stem cells, wherein the embedded or coated cells are allowed sufficient time for in-growth of the patient's cells into the scaffold prior to implanting, transplanting, or administering the scaffold to the tissue.

In carrying out a method of the invention, a scaffold or graft is prepared as described herein, or other known method of preparing ECM, then implanted, transplanted, or administered to at least a section of tissue which is diseased or damaged, and allowed to remain in contact with the diseased or damaged tissue for a sufficient period of time so that replacement cells grow and replace the diseased or damaged cells. A sufficient period of time can be from one day to about six months.

Advantageously, the implantation, transplantation, or administration of an ECM graft provides a stimulus for migration of stem cells to the site of implantation, transplantation or administration of the ECM graft. For example, in treating UC, an ECM graft is implanted, transplanted or administered in contact with at least a section of the colon where the diseased or damaged colon mucosa was (in the case of post-mucosectomy) or is (in the case where mucosectomy is not preformed), retained in contact with the mucosa for a period of time to allow growth and replacement of colon mucosal cells, resulting in replacement of the diseased or damaged cells with healthy cells. The ECM graft can be provided as a relatively short solid segment, on the order of 1-10 centimeters, concomitantly or sequentially implanted, transplanted or administered to the inner wall of the colon. Alternatively, the ECM graft can be prepared having a plurality of sections or lengths corresponding to the entire colon.

### BRIEF DESCRIPTION OF THE FIGURES

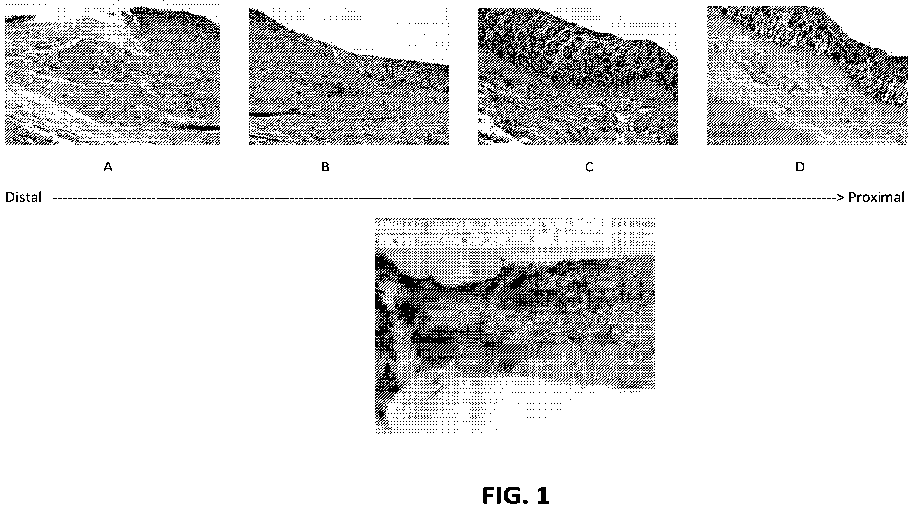

FIG. 1 shows the results of a colon section (lower photograph) removed from a dog treated with Small Intestinal Submucosa Extracellular Matrix (SIS-ECM) as described herein; micrographs (upper photographs A-D) show microscopic cellular examinations along the distal → proximal gradient of the colon section illustrating an increased mucosal coverage along the gradient, namely, incomplete mucosal coverage is seen at the distal anastomosis (A), and increasing mucosal coverage is present toward the proximal ends (B), (C), and (D).

### DETAILED DESCRIPTION OF THE INVENTION

The subject invention concerns treating Inflammatory Bowel Disease (IBD), for example Ulcerative Colitis (UC) or Crohn's Disease (CD), or the like, by replacing or stimulating replacement of diseased or damaged mucosal cells and tissue without colectomy.

The method of the invention as defined in claim 1, comprises treating damaged or diseased lower intestinal tissue by the steps of (1) optionally performing a mucosectomy on the diseased or damaged mucosal lining of the colon, and (2) seeding the mucosal lining of the colon with stem or progenitor cells, or stimulating stem and progenitor cell migration to the diseased or damaged colon tissue in a patient having symptoms of, or suffering from, disease or damage to the mucosal lining of the colon. A disease of the mucosal lining of the colon can be Inflammatory Bowel Disease (IBD), and the method can be used to treat, ameliorate, or cure IBD.

In one embodiment of the invention, stem cells or progenitor cells can be stimulated to the site of the damaged or diseased colon tissue by introducing onto, or in contact with, the colon luminal wall an extracellular matrix (ECM) composition, e.g., an ECM graft or scaffold, in the form of a solid (sheet or tube) or fluid (solution, suspension, or gel).

In another embodiment of the invention, stem cells or progenitor cells can be incorporated into the solid or fluid ECM composition, and the ECM composition comprising stem cells or progenitor cells can be delivered to the mucosal lining of the colon by delivery means appropriate for the composition as described herein. For example, a solid ECM can be endoscopically implanted onto the colon lining, or a fluid ECM can be infused, injected, or sprayed into the colon.

By design, ECM breaks down rapidly in situ and encourages an inflammatory response, including migration of macrophages to the site. One unique characteristic of ECM is to encourage a switch in the phenotype of these macrophages from type M1 (pro-inflammatory, such as would be seen in a patient suffering from UC) to type M2 (pro-tissue remodeling, such as would be seen in a person without an underlying pro-inflammatory condition). An important ramification of this M1 to M2 phenotype switch is to allow tissue that is otherwise predisposed to inflammation to remain healthy.

The optional mucosectomy step, which is carried out to remove diseased or damaged tissue, e.g., colon mucosal tissue, prior to implantation, transplantation, or administration of a graft, can be performed using any commonly accepted method, such as tissue resection or ablation. Known resection or ablation methods include endoscopic or surgical mucosal resection (excision), endoscopic laser therapy, photodynamic therapy, endoscopic thermocoagulation using argon plasma coagulation, cryotherapy, radiofrequency ablation or ablation using EDTA or other ablating agent, such as heated or "hot" saline or other aqueous solution.

The stem cells are introduced to a mucosectomized tissue by placing and/or securing, e.g., implanting, transplanting, or administering, extracellular matrix (ECM) material or composition as an ECM scaffold or graft at the site. Introduction of an ECM composition to the mucosectomized site can be in the form of a solid sheet or tube, or can be in the form of a fluid, such as a solution, suspension or gel. The placement or securing of the solid ECM material can be by sutures, clips, staples, glue, or other securing means as is well known.

Alternatively, a fluidized ECM can be administered by a commonly employed fluid administration or delivery process such as injection, infusion or drench ("squirting" onto the site) or by spraying the fluid onto the site. A preferred spraying technique is carried out using an aerosolized ECM fluid, delivered, for example endoscopically.

As used herein, the terms "composition," "material," "scaffold," and "graft," as referring to ECM, can be used interchangeably, and do not connote a particular configuration, such as being in solid, liquid, fluid or other form. For example, an "ECM scaffold" can be solid or fluid.

Preferably, a fluidized ECM composition is formed as a gel such that it has viscous properties, and has a viscosity sufficient so that the fluid is self-adhering to the desired location within the body. In a preferred embodiment, the fluidized ECM is a hydrogel having relatively non-viscous liquid properties when applied or administered, which advantageously thickens or becomes more viscous, forming an adhesive gel upon, or shortly following, contact with the body at the site of administration or application. Gelation can be initiated by increased temperature, such as body heat following administration. The fluid ECM can also be mixed with a separate viscous agent, such as a hydrogel or other commonly known gelling agent to provide sufficient viscosity. Alternatively, two fluids can be administered whereby at least one of the fluid contains ECM material, and wherein the two fluids gel when coming into contact with one another or when mixed.

It is well established that an ECM composition can cause stem cells to migrate to the site when sufficient vascularization of the tissue exists, and the blood vessels provide an adequate conduit for the cells to migrate to the site. In addition, ECM communicates with the adjacent or underlying tissue via chemical signaling, and the adjacent or underlying tissue communicates with the ECM, by a phenomenon termed "dynamic reciprocity" which optimizes cell and tissue replacement or regeneration where ECM is used. Thus, the ECM composition can serve as a solid, liquid or gel scaffold for new cellular and tissue growth at the site of placement, administration, or application.

In a further embodiment, an ECM-derived composition can be a tissue graft whereby the ECM composition serves as a scaffold for promoting new growth of tissue at the site of implant. The scaffold can be an ECM tube or sheet placed at the desired site. The scaffold can be a biocompatible porous, macroporous, or microporous matrix, wherein stem cells or ECM gel, solution or suspension can be dispersed. The ECM can also be subsequently gelled following dispersion of the stem cells or ECM solution or suspension.

The ECM is derived from small intestinal submucosa (SIS).

As used herein, the terms implanting or implantation, transplanting or transplantation, applying or application, administering or administration, infusing or infusion, injecting or injection, delivering or delivery, all refer to the process or providing an ECM to the site of treatment, and would be understood by a person of ordinary skill in the art to have the same meaning, depending on the composition properties and procedure employed for carrying out the delivery of ECM to the site. These terms can be used interchangeably and are in no way limiting to the method of the invention.

Optionally, when treating the colon, the method can further include one or more of the additional steps: (3) creating an ileostomy, and (4) providing Total Parenteral Nutrition (TPN) to the patient by feeding the patient intravenously while the new mucosa forms.

In a preferred embodiment, this method comprises treating damaged or diseased colon tissue. The method can be employed, for example, to treat damage to or disease of colon tissue resulting from Crohn's Disease, Ulcerative Colitis, familial adenomatous polyposis, Hirschsprungs, stricture, proctitits, or fistula, and more preferably, treating disease or damage to colon mucosa tissue.

Methods for preparing tube, sheet, and gelled, solubilized ECM compositions, useful as cell growth scaffolds, are described in the art. Gel ECM compositions can be molded prior to implantation or administered to a patient in an un-gelled form prior to gelation where the composition gels in situ.

These, and other methods of forming ECM grafts are described in, for example, US Patent No. 8,361,503. In a preferred embodiment, a gel ECM composition is prepared according to one or more processes as described. For example, a gel ECM can be prepared by a method comprising:
i. comminuting an ECM,
ii. solubilizing intact, non-dialyzed or non-cross-linked ECM by digestion with an acid protease in an acidic solution to produce a digest solution,
iii. adjusting the pH of the digest solution to a pH between 7.2 and 7.8 to produce a neutralized digest solution, and
iv. gelling the solution at a temperature greater than 25 °C.

In another embodiment the method of preparing an ECM scaffold further includes ultrasonicating the scaffold.

In carrying out a method of the invention, a scaffold graft is prepared in accordance with the description herein, then implanted, transplanted, or administered to at least a section of tissue which is diseased or damaged, and allowed to remain in contact with the diseased or damaged tissue for a sufficient period of time so that replacement cells grow and replace the diseased or damaged cells.

A sufficient period of time can be from one day to a few weeks or months, depending on the responsiveness of the patient to such treatment method. A one-year period of treatment without successful tissue reconstruction may be an upper limit for continuing such treatment. The ECM material biodegrades naturally in the body and is not required to be removed.

For carrying out the subject method treating UC, an ECM graft is implanted, transplanted or administered in contact with at least a section of diseased or damaged colon, retained in contact with the mucosa for a period of time to allow growth and replacement of colon mucosal cells, resulting in replacement of the diseased or damaged cells with healthy mucosal cells that do not exhibit inflammation or other manifestations of UC-diseased cells.

The ECM graft can be provided as a relatively short tube or sheet segment, on the order of 1-10 centimeters in length, implanted, transplanted or administered to the inner wall of the colon. Alternatively, the ECM graft can comprise one or more lengths which correspond to the entire length of the colon. A solid tube or sheet ECM can be secured to the tissue to be treated by suturing, clips, staples, glue, or the like. Alternatively, the colon tissue can be treated by administering, e.g., spraying, a liquid solution, suspension, or gel composition onto the site being treated.

In a method of treating UC that includes a mucosectomy step, the tissue exhibiting the symptoms of UC is removed, since UC is confined within the mucosa. The mucosectomy step can be performed endoscopically, obviating the need for open or laparoscopic surgery. The stem cell stimulation step allows a new, healthy mucosa to form in the colon lumen. The mucosectomy can be done to a partial or short segment of the colon or longer lengths of the colon.

Whether carried out on a segment of the colon or the entire length of the colon, the subject invention can offer benefits which eliminate the need for abdominal colectomy and leaving a native rectal tissue cuff, in situ, including the benefits of removing the malignant potential in the retained rectum from proliferative (malignant) pathologies, e.g., UC or familial polyposis; and allowing a healthcare professional to leave a longer rectosigmoid segment in place, with functional benefits of:
- Decreased risk of sphincter disruption (incontinence) or sacral nerve damage (impotence) that come with lower dissections, and
- Improved reservoir function, allowing more normal bowel habits because of increased water absorption and improved storage capacity;
- Offers relative simplicity and less invasiveness than colectomy or similar procedures such as IPAA (total proctocolectomy with ileoanal anastomosis); a mucosectomy is a fairly straightforward procedure which can be done from below at same time as the pull-through/ileostomy or at any time later. The stem cells could be "planted" and regenerate the mucosa while the diverting ileostomy is still extant.
- Sparing the colon obviates the lifelong concerns of living without a colon (e.g. pouchitis, intestinal blockage, dehydration, etc.);
- Creates only a temporary Ileostomy (i.e., until the new mucosa is viable); and
- It brings together various disciplines (surgical, medical, endoscopic, and tissue engineering) that might otherwise work independently to treat IBD.

It would be understood that various methods and procedures can be used to deliver stem cells to the colon, e.g. on a scaffold, in a broth delivered via enema, by a catheter-based delivery system, or the like. Advantageously, the ECM material can be applied during the same surgical procedure as when the ileostomy is created, and the mucosa can be allowed to proliferate or regenerate while the internal pouch heals.

### EXAMPLE 1 - Surgical placement of solid ECM for replacement of colon tissue

Small intestine submucosal extracellular matrix (SIS-ECM) grafts were grafted into the colon tissue of four (4) living dogs. The SIS-ECM grafts of approximately 3-4 centimeter lengths were prepared in accordance with known techniques. Grafts were prepared having different numbers of layers - 2-layer, 4-layer, 6-layer, and 8-layer sheets or tubes and tested following transanal circumferential mucosal resection.

Four (4) healthy adult mongrel female dogs (approx. 20 kg in weight) were subjected to circumferential colon mucosal EMR. With the dog under general anesthesia, surgical mucosectomy was performed. Alternatively, mucosal tissue ablation, such as EMR can be performed, e.g., with a therapeutic endoscope (EG-3430, Pentax Medical, Montvale, NJ) and a commercially available kit (EMR Kit, Olympus America, Center Valley, Pa). Piecemeal mucosal resections were sequentially performed until a 4-cm circumferential resection was completed.

A tubular ECM graft or scaffold derived from the porcine small intestine submucosa (SIS) was then endoscopically surgically placed in the four dogs. The SIS-ECM biologic scaffold material was prepared as previously described and configured into a tubular shape. In brief, porcine SIS was harvested from market-weight pigs (approximately 110-130 kg) immediately after death. Residual external connective tissues, including adipose tissue, were trimmed and all residual waste material was removed by repeated washes with tap water. The submucosal layer was mechanically delaminated from the small intestine tissue. The submucosal layer was then decellularized and disinfected by immersion in 0.1% (vol/vol) peracetic acid (s), 4% (vol/vol) ethanol, and 96% (vol/vol) deionized water for 2 hours.

The SIS-ECM material was then washed twice for 15 minutes with phosphate-buffered saline solution (pH Z 7.4) and twice for 15 minutes with deionized water. Tubular scaffolds were fabricated to match the anatomy of the canine colon. Briefly, multilayer tubes were created by wrapping hydrated sheets of SIS around a 22-mm perforated tube/mandrel that was covered with umbilical tape for a total of several complete revolutions (i.e., a multi-layer tube). The constructs were then placed into plastic pouches and attached to a vacuum pump (model D4B, Leybold, Export, PA) with a condensate trap in line.

The constructs were subjected to a vacuum of 710 to 740 mm Hg for 10 to 12 hours to remove the water and form a tightly coupled multi-laminate construct. After each ECM device was removed from the mandrel, they were terminally sterilized with ethylene oxide.

For endoscopic placement of the SIS-ECM graft, the tubular scaffold was hydrated in a saline solution bath for 5 minutes and then placed over a 30-mm achalasia balloon (Cook Endoscopy Achalasia balloon, Wilson-Cook Medical, Winston-Salem, NC). The SIS-ECM device was constrained with two 4-0 silk sutures with surgeon's knots that would release when the balloon was inflated. A 0.035-inch wire (Jagwire, Boston Scientific, Natick, MA) was endoscopically placed into the dog's colon. The balloon was then passed over the wire and positioned under endoscopic guidance with the SIS-ECM bridging the length of the mucosal resection.

One ml of a degradable, lysine-derived urethane (LDU) surgical adhesive (TissuGlu, Cohera Medical, Pittsburgh, Pa) was then injected through a 6F endoscopic guiding catheter (Oasis stent introduction system, Wilson-Cook Medical) between the colon wall and the SIS-ECM in 2 separate strips on opposite sides of the device to prevent slippage. The balloon was then manually inflated to full capacity, expanding the scaffold against the colon wall. Balloon inflation was maintained for 15 minutes before deflation and removal, leaving the SIS-ECM scaffold in place within the colon.

Postoperatively, the dogs were recovered from anesthesia, extubated, and monitored in the recovery room until they were resting comfortably in a sternal position. The dogs were kept in a cage overnight and returned to their larger run housing on postoperative day 1. All dogs were given oral prophylactic antibiotics consisting of cephalothin/cephalexin (35 mg/kg) twice daily for 7 to 9 days.

Intravenous acepromazine (0.1 mg/kg) and butorphanol (0.05 mg/kg) were administered for 2 days, followed by subcutaneous or intramuscular buprenorphine (0.01 to 0.02 mg/kg) every 12 hours thereafter as needed for analgesia.

All dogs were also given omeprazole 20 mg daily. Oral intake began 36 hours after surgery. Dogs were fed from an elevated/raised platform. Daily nutritional requirements were calculated and divided into 3 separate feedings. Gruel/soft food was provided for 1 week postoperatively followed by a gradual change to solid food over the ensuing 2-week period. The dogs were weighed weekly and housed in a run measuring approximately 10-14 feet to allow freedom to ambulate.

Endoscopic examinations were conducted 1 month postoperatively and immediately preceding euthanasia at 12 weeks to evaluate colon mucosal appearance and stricture.

Immediately after euthanasia, the scaffold placement site and the native colon tissue proximal and distal to the scaffold placement site were harvested. The excised segment was split longitudinally and the exposed mucosal surface was examined and photographed for dimensional measurements. The luminal circumference of the colon was measured 3 cm proximal to the superior edge of the remodeled site and in the middle of the graft to determine the extent of stenosis. Results were expressed as percent reduction of the circumference between the remodeled site and the proximal normal tissue (mean +/- SD).

The excised tissue was pinned to corkboard in a flattened position and immersed in 10% neutral buffered formalin. The specimen was trimmed longitudinally including both normal and remodeled tissue, sectioned, and stained with both hematoxylin-eosin and Masson's trichrome stains. The areas examined included the native tissue, the proximal and distal interfaces between the remodeled area and the native tissue, and the middle region of the remodeled area. On the basis of examination of the distributions of data, the data appear to be normally distributed. Thus, the statistical approach used to compare the results was the parametric t test (n = 4).

The hypothesis tested was that the treatment of the EMR defect with SIS-ECM would cause less reduction in the circumference compared with no treatment. It is recognized that data from individual test animals were subjected to multiple statistical analyses (i.e., circumference reduction and weight.) The comparison of reduction in luminal circumference in the dogs was taken as the primary statistical analysis, which did not involve multiple testing. All other statistical tests are considered to be secondary with their P values stated uncorrected for repeated measures and should be taken as descriptive only.

### RESULTS AND CONCLUSIONS.

In the absence of treatment, scarring and stricture formation is expected outcome. However, ECM treatment resulted in mucosal coverage of the resected tissue that appeared normal grossly and microscopically. Outcomes varied slightly based on number of ECM layers in the tubular device - higher numbers of ECM layers were generally associated with increased mucosal coverage. No signs of stricture were present in any dogs treated with an ECM scaffold.

The current study in a dog model showed that a SIS-ECM scaffold, deployed endoscopically after circumferential EMR, facilitated colon mucosal remodeling (FIG. 1) without stricture formation. The remodeled tissue consisted of a completely epithelialized lumen with a dense, organized collagenous submucosa and normal-appearing muscularis externa.

Thus, these studies using a dog model and employing SIS-ECM grafts for regeneration of colon mucosal lining tissue demonstrate that ECM can be used in treating IBD, such as UC or CD.

Having thus described the invention it is clear that what may appear to be different embodiments could be provided without departing from the spirit and scope of the invention. Hence it is intended that the foregoing specification be interpreted as illustrative rather than in a limiting sense.

### EXAMPLE 2 - Application of gel ECM for replacement of colon tissue

In accordance with the invention, the feasibility of using an ECM gel composition for treatment of Inflammatory Bowel Disease (IBD) can be tested *in vivo* in rats. Rats may be established as a model for human IBD, such as Ulcerative Colitis (UC), whereby UC can be induced in rat colon tissue by administering Dextran Sulfate Sodium (DSS) in drinking water ad libitum for several days.

Acute UC can be induced in rats by administering, in drinking water provided ad libitum, 2% DSS for seven days, followed by a saline flush. Chronic UC can be induced in rats by administering, in drinking water provided ad libitum, 2% DSS for one or more consecutive 7-day periods, followed by administration of regular water.

Treatment of UC can be carried out using a gel extracellular matrix (ECM) derived from small intestine submucosa (SIS) and introduced into the colon via enema. The gel SIS-ECM can be administered one or more times-per-day for a period of at least one week, and preferably up to about one month. A preferred dosing regimen for SIS ECM is once per day for 30 days.

### Method of Preparation of Gels from ECM

The preparation of SIS from a segment of small intestine is detailed in U.S. Pat. No. 4,902,508, US Pat. No. 5,275,826, and US Pat. No. 5,514,533. A segment of intestine is first subjected to abrasion using a longitudinal wiping motion to remove both the outer layers (particularly the tunica serosa and the tunica muscularis) and the inner layers (the luminal portions of the tunica mucosa). Typically the SIS is rinsed with saline and optionally stored in a hydrated or dehydrated state until use as described below.

The present fluidized compositions are prepared as solutions or suspensions of intestinal submucosa by comminuting and/or digesting the submucosa with a protease, such as trypsin or pepsin, for a period of time sufficient to solubilize said tissue and form a substantially homogeneous solution. The intestinal submucosa starting material is comminuted by tearing, cutting, grinding, shearing and the like. Grinding the submucosa in a frozen or freeze-dried state is preferred although good results can be obtained as well by subjecting a suspension of pieces of the submucosa to treatment in a high speed (high shear) blender and dewatering, if necessary, by centrifuging and decanting excess water. The comminuted intestinal submucosa can be dried to form a submucosa powder. Thereafter, it can be hydrated, that is, combined with water or buffered saline and optionally other pharmaceutically acceptable excipients to form a tissue graft composition as a fluid having a viscosity of about 2 to about 300,000 cps at 25°C. The higher viscosity graft compositions can have a gel or paste consistency. The present compositions can be sterilized using art-recognized sterilization techniques such as exposure to ionizing radiation.

The fluidized submucosa of this invention also finds use as an injectable heterograft for tissues, for example, soft tissues, in need of repair or augmentation most typically to correct trauma or disease-induced tissue defects.

### SIS Suspension

SIS specimens prepared as described above are minced or chopped into arbitrarily small pieces using tissue scissors, a single-edged razor blade, or other appropriate cutting implement. The specimens are placed in a flat bottom stainless steel container and liquid nitrogen is introduced into the container to freeze the specimens to prepare them for comminuting.

The frozen SIS specimens are then comminuted to form a coarse SIS powder. Such processing can be carried out, for example, with a manual arbor press with a cylindrical brass ingot placed on top of the frozen specimens. The ingot serves as an interface between the specimens and the arbor of the press. Liquid nitrogen can be periodically added to the SIS specimens to keep them frozen.

Other methods for comminuting SIS specimens may be utilized to produce an SIS powder usable in accordance with the present invention. For example, SIS specimens can be freeze-dried and then ground using a manual arbor press or other grinding means. Alternatively, SIS can be processed in a high shear blender to produce, upon dewatering and drying, an SIS powder.

Further grinding of the SIS powder using a pre-chilled mortar and pestle can be used to produce consistent, more finely divided product. Again, liquid nitrogen is used as needed to maintain solid frozen particles during final grinding. The powder can be easily hydrated using, for example, buffered saline to produce a fluidized tissue graft material of this invention at the desired viscosity.

### SIS Solution

SIS powder is sifted through a wire mesh into any convenient vessel. The powder is then subjected to proteolytic digestion to form a substantially homogeneous solution. In one embodiment, the powder is digested with 1 mg/ml of pepsin (Sigma Chemical Co., St. Louis, Mo.) in 0.1 M acetic acid, adjusted to pH 2.5 with HCl, over a 48 hour period at room temperature. The reaction medium is neutralized with sodium hydroxide to inactivate the peptic activity. The solubilized submucosa may then be concentrated by salt precipitation of the solution and separated for further purification and/or freeze drying to form a protease solubilized intestinal submucosa in powder form.

The viscosity of fluidized submucosa compositions in accordance with this invention can be manipulated by controlling the concentration of the submucosa component and the degree of hydration. The viscosity can be adjusted to a range of about 2 to about 300,000 cps at 25°C. Low viscosity submucosa compositions are better adapted for intra-articular applications or applications within body cavities. Higher viscosity formulations, for example, gels, can be prepared from the SIS digest solutions by adjusting the pH of such solutions to about 6.0 to about 7.0. Gel forms of the present compositions, as submucosa suspensions or submucosa digest solutions, are typically preferred for subcutaneous or intramuscular applications using syringes or catheters.

SIS gel has also been described as being formed into a gel by mixing 0.1 N NaOH (1/10 of the volume of digest solution) and 10X PBS pH 7.4 (1/9 of the volume of digest solution) in appropriate amounts at 4° C. The solution was brought to the desired volume and concentration using cold (4°C.) 1X PBS pH 7.4 and placed in a 37°C incubator for gelation to occur.

The ECM was able to form a matrix after 40 minutes in solution. The ECM-derived gel was liquid at temperatures below 20°C. but turns into a gel when the temperature is raised to 37°C.

In preparing gels from ECM, all of the solutions should be kept on ice and the following variables must be determined in accordance with US Pat. No. 8,361,503:
C_{f} = concentration of the final gel in mg/ml
Cs = concentration of the ECM digest solution in mg/ml
V_{f} = volume of the final gel solution needed for the experiments
V_{d} = volume needed from the ECM digest solution in ml
V₁₀ₓ = volume of 10X PBS needed in ml
V₁ₓ = volume of 1X PBS needed in ml
V_{NaOH} = volume of 0.1 N NaOH needed in ml

First, determine the final concentration (C_{f}) and volume (V_{f}) of ECM gel required. Then, calculate the mass of ECM needed by multiplying C_{f} (mg/ml)* V_{f} (ml). This value will give you the volume needed from the ECM digest solution (V_{d}), where V_{d} = [C_{f}(mg/ml)* V_{f}(ml)]/Cₛ.

Calculate the volume of 10X PBS needed by dividing the calculated volume V_{d} by 9 (V_{10X} = V_{d} /9). Calculate the volume of 0.1 N NaOH needed by dividing the calculated volume V_{d} by 10 (V_{NaOH} = V_{d} /10). Calculate the amount of 1X PBS needed to bring the solution to the appropriate concentration/volume as follow: V_{1X}=V_{f}-V_{d}-V_{10X}-V_{NaOH}. Add all the reagents (V_{1X} + V_{d} + V_{10X} + V_{NaoH}) to an appropriate container (usually 15 or 50 ml centrifuge tubes) without the ECM digest (V_{d}). Place solutions on ice and keep on ice at all times.

Add the appropriate volume from the ECM digest solution (V_{d}) to the PBS/NaOH mixture prepared above and mix well with a 1 ml micropipette while being careful and avoiding the creation of air bubbles in the solution. Depending on the viscosity of the ECM digest solution, there might be some significant volume loss during the transfer. Monitor the total volume and add appropriate amounts until the final volume is achieved. Measure the pH of the pre-gel solution, where pH should be around 7.4.

Add the pre-gel solution to a mold or to appropriate wells. Place the mold or wells in a 37°C incubator for a minimum of 40 minutes. Avoid using an incubator with CO₂ control. If water evaporation is a concern, place the mold inside a plastic zip-lock bag before placing in the incubator. After gelation, the gel can be removed from the mold and placed on 1X PBS. If the gels were made in tissue culture plates, 1X PBS can be placed on top of the gels until use to maintain the gels hydrated. Sample calculation: Make 6 ml of gel with a final concentration of 6 mg/ml from the 10 mg/ml stock solution.

### SIS-ECM Administration procedure

SIS-ECM solution or suspension can be administered by enema into the colon of the UC-induced rats. No rejection, infection, or abnormal physiologic response of the host animal is expected following administration of the graft. The solution or suspension may also be administered via endoscopy and via laparoscopy into the colon. It is believed that an unexpected result of the current invention will be stimulation of appropriate tissue remodeling such that augmentation of colon mucosa can be accomplished with SIS solution or suspension material.

The fluidized compositions of this invention can result in tissue replacement and repair, and further result in treatment or cure of IBD, including UC. The fluidized submucosal compositions are used in accordance with the present method to induce regrowth of natural colon mucosal tissue. By injecting an effective amount of a fluidized ECM composition into the locale of the defective tissue, the biotropic properties can be realized without the need for more invasive surgical techniques.

Although the invention has been described in detail with reference to certain preferred embodiments, variations and modifications exist within the scope defined in the following claims.

## Claims

1. A composition for use in treating, ameliorating, or curing a condition or disease of lower intestinal tract tissue of a vertebrate, or damage to said tissue, said composition comprising an extracellular matrix (ECM) graft or scaffold derived from small intestinal submucosa (SIS) for delivering or stimulating migration of stem cells or progenitor cells to the diseased or damaged tissue, to promote reconstructive tissue remodeling of the diseased or damaged tissue to effect the treatment, amelioration, or cure of the diseased or damaged tissue without colectomy.

2. The composition for the use according to claim 1 wherein the composition is prepared for delivery as a fluid selected from a solution, suspension, and gel.

3. The composition for the use according to claim 1, wherein the composition is prepared for delivery as a solid sheet or tube.

4. The composition for the use according to claim 2, wherein the composition is formulated for endoscopical delivery or delivery by enema.

5. The composition for the use according to any one of claims 1 to 4, wherein the composition further comprises a drug or biologic.

6. The composition for the use according to any one of claims 1 to 5, wherein the lower intestinal tissue is diseased or damaged as a result of Crohn's Disease, Ulcerative Colitis, familial adenomatous polyposis, Hirschsprungs, stricture, proctitits, colon or rectal cancer, or fistula.

7. The composition for the use according to any one of claims 1 to 6, wherein the composition is applied by implanting, transplanting, administering, applying or adhering or affixing said composition onto the diseased or damaged lower intestinal tract tissue, whereby the condition, disease, or damage to the lower intestinal tract tissue is treated, ameliorated, or cured by reconstructive tissue remodeling without colectomy.

8. The composition for the use according to any one of claims 1 to 7, wherein the tissue exhibiting the condition, disease, or damage is colon.

9. The composition for the use according to any one of claims 1 to 8, wherein said composition is applied in a vertebrate following resection or ablation of the tissue in the lower intestinal tract exhibiting the condition, disease, or damage.

10. The composition for the use according to claim 9, wherein the resection or ablation comprises endoscopic mucosal resection or EDTA wash.

## Patentansprüche

1. Eine Zusammensetzung zur Verwendung bei der Behandlung, Verbesserung oder Heilung eines Zustandes oder einer Erkrankung von Gewebe des unteren Verdauungstraktes eines Wirbeltieres oder einer Schädigung besagten Gewebes, wobei die Zusammensetzung ein extrazelluläres Matrix (ECM)-Transplantat oder -Gerüst umfasst, das von einer Dünndarmsubmukosa (SIS) abgeleitet ist, zur Abgabe oder Stimulierung der Migration von Stammzellen oder Vorläuferzellen zu dem erkrankten oder geschädigtem Gewebe, um die rekonstruktive Geweberemodellierung des erkrankten oder geschädigten Gewebes zu erleichtern, um die Behandlung, Verbesserung oder Heilung des erkrankten oder geschädigten Gewebes ohne Kolektomie zu bewirken.

2. Die Zusammensetzung zur Verwendung nach Anspruch 1, wobei die Zusammensetzung zur Verabreichung als Flüssigkeit, ausgewählt aus einer Lösung, Suspension und Gel, hergestellt ist.

3. Die Zusammensetzung zur Verwendung nach Anspruch 1, wobei die Zusammensetzung zur Verabreichung als feste Platte oder Rohr hergestellt ist.

4. Die Zusammensetzung zur Verwendung nach Anspruch 2, wobei die Zusammensetzung zur endoskopischen Verabreichung oder zur Verabreichung durch einen Einlauf formuliert ist.

5. Die Zusammensetzung zur Verwendung nach einem der Ansprüche 1 bis 4, wobei die Zusammensetzung ferner ein Arzneimittel oder ein biologisches Mittel umfasst.

6. Die Zusammensetzung zur Verwendung nach einem der Ansprüche 1 bis 5, wobei das untere Darmgewebe aufgrund von Morbus Crohn, Colitis ulcerosa, familiärer adenomatöser Polyposis, Hirschsprung, Striktur, Proktites, Darm- oder Rektumkarzinom oder Fistel erkrankt oder geschädigt ist.

7. Die Zusammensetzung zur Verwendung nach einem der Ansprüche 1 bis 6, wobei die Zusammensetzung durch Implantieren, Transplantieren, Verabreichen, Auftragen oder Anhaften oder Befestigen der Zusammensetzung auf das erkrankte oder geschädigte Gewebe des unteren Darmtraktes aufgebracht wird, wobei der Zustand, die Erkrankung oder die Schädigung des Gewebes des unteren Darmtraktes durch rekonstruktive Geweberemodellierung ohne Kolektomie behandelt, verbessert oder geheilt wird.

8. Die Zusammensetzung zur Verwendung nach einem der Ansprüche 1 bis 7, wobei das Gewebe, das den Zustand, die Krankheit oder die Schädigung aufweist, Dickdarm ist.

9. Die Zusammensetzung zur Verwendung nach einem der Ansprüche 1 bis 8, wobei die Zusammensetzung in einem Wirbeltier nach Resektion oder Ablation des Gewebes im unteren Darmtrakt, das den Zustand, die Krankheit oder die Schädigung aufweist, angewendet wird.

10. Die Zusammensetzung zur Verwendung nach Anspruch 9, wobei die Resektion oder Ablation eine endoskopische Schleimhautresektion oder Waschung mit EDTA umfasst.

## Revendications

1. Composition destinée à être utilisée pour traiter, améliorer ou guérir une condition ou une maladie du tissu du tractus intestinal inférieur d'un vertébré ou un endommagement dudit tissu, ladite composition comprenant un greffon ou un échafaudage de matrice extra-cellulaire (MEC) dérivé d'une petite sous-muqueuse d'intestin grêle (SIS) pour faciliter le remodelage tissulaire reconstructif dudit tissu malade ou endommagé pour effectuer le traitement, l'amélioration ou la guérison dudit tissu malade ou endommagé sans colectomie.

2. Composition pour l'utilisation selon la revendication 1, la composition étant préparée pour être administrée sous forme de fluide choisi parmi une solution, une suspension et un gel.

3. Composition pour l'utilisation selon la revendication 1, la composition étant préparée pour être administrée sous la forme d'une feuille solide ou d'un tube solide.

4. Composition pour l'utilisation selon la revendication 2, la composition étant formulée pour une administration endoscopique ou une administration par lavement.

5. Composition pour l'utilisation selon l'une quelconque des revendications 1 à 4, la composition comprenant en outre un médicament ou un produit biologique.

6. Composition pour l'utilisation selon l'une quelconque des revendications 1 à 5, le tissu intestinal inférieur étant malade ou endommagé du fait de la maladie de Crohn, de la colite ulcéreuse, de la polypose adénomateuse familiale, de la maladie de Hirschsprung, de la sténose, de la proctite, des cancers du côlon ou du rectum ou de la fistule.

7. Composition pour l'utilisation selon l'une quelconque des revendications 1 à 6, la composition étant appliquée par implantation, transplantation, administration, application ou adhérence ou fixation de ladite composition sur le tissu du tractus intestinal inférieur malade ou endommagé, la condition, la maladie ou l'endommagement du tissu du tractus intestinal inférieur étant traités, améliorés ou guéris par un remodelage tissulaire reconstructif sans colectomie.

8. Composition pour l'utilisation selon l'une quelconque des revendications 1 à 7, dans lequel le tissu présentant la condition, la maladie ou l'endommagement est le côlon.

9. Composition pour l'utilisation selon l'une quelconque des revendications 1 à 8, ladite composition étant appliquée chez un vertébré par suite d'une résection ou d'une ablation du tissu dans le tractus intestinal inférieur présentant la condition, la maladie ou l'endommagement.

10. Composition pour l'utilisation selon la revendication 9, la résection ou l'ablation comprenant une résection muqueuse endoscopique ou un lavage à l'EDTA.
